# EUROPEAN PATENT APPLICATION

(11) **EP 4 450 994 A1**
(43) Date of publication of application: **23.10.2024**
(21) Application number: 23168889.6
(22) Date of filing: 20.04.2023
(51) Int. Cl.: G01R 33/54, G16H 30/40, A61B 5/055, G16H 40/63

(54) **AUTOMATED CONFIGURATION OF MAGNETIC RESONANCE IMAGING SYSTEMS**

(71) Applicant: Koninklijke Philips N.V., 5656 AG Eindhoven (NL)
(72) Inventor: HAHN, Artur, Eindhoven (NL); MEINEKE, Jan Jakob, Eindhoven (NL); KEUPP, Jochen, 5656AG Eindhoven (NL); BOERNERT, Peter Ulrich, Eindhoven (NL)
(74) Representative: Philips Intellectual Property & Standards

(57) **Abstract**

Disclosed herein is a medical system (100, 300, 500) comprising a memory (116) storing machine executable instructions (120) and a graph database (122). The graph database is configured to output magnetic resonance imaging pulse sequence configuration data (126) in response to receiving subject data. The medical system further comprises a computational system (110). Execution of the machine executable instructions causes the computational system to: receive (200) the subject data; and receive (202) the magnetic resonance imaging pulse sequence configuration data in response to inputting the subject data into the graph database.

## Description

### FIELD OF THE INVENTION

The invention relates to magnetic resonance imaging, in particular to the configuration of magnetic resonance imaging systems.

### BACKGROUND OF THE INVENTION

A large static magnetic field is used by Magnetic Resonance Imaging (MRI) scanners to align the nuclear spins of atoms as part of the procedure for producing images within the body of a patient. This large static magnetic field is referred to as the B0 field. Configuring magnetic resonance imaging systems can be complicated and may require years of training.

The journal article Deneck et. al., "Automated Protocoling for MRI Exams-Challenges and Solutions," Journal of Digital Imaging (2022) 35: 1293-1302, https://doi.org/10.1007/s10278-022-00610-1 discloses that automated protocoling for MRI examinations is an amendable target for workflow automation with artificial intelligence. However, there are still challenges to overcome for a successful and robust approach. A literature review was performed that analyzed limitations of currently published approaches for automated protocoling. It assessed these limitations quantitatively based on data from a private radiology practice. For this, it was further assessed the information content provided by the clinical indication by computing the overlap coefficients for the sets of ICD-10-coded admitting diagnoses of different MRI protocols. Additionally, it was assessed the heterogeneity of protocol trees from three different MRI scanners based on the overlap coefficient, on MRI protocol and sequence level. Additionally, a sequence name standardization was applied to demonstrate its effect on the heterogeneity assessment, i.e., the overlap coefficient, of different protocol trees. The overlap coefficient for the set of ICD-10-coded admitting diagnoses for different protocols ranges from 0.14 to 0.56 for brain/head MRI exams and 0.04 to 0.57 for spine exams. The overlap coefficient across the set of sequences used at two different scanners increases when applying sequence name standardization (from 0.81/0.86 to 0.93). Automated protocoling for MRI examinations has the potential to reduce the workload for radiologists.

### SUMMARY OF THE INVENTION

The invention provides for a medical system, a computer program, and a method in the independent claims.

In one aspect the invention provides for a medical system that comprises a memory storing machine-executable instructions and a graph database. The graph database is configured to output magnetic resonance imaging pulse sequence configuration data in response to receiving subject data. The medical system further comprises a computational system. Execution of the machine-executable instructions causes the computational system to receive the subject data. Execution of the machine-executable instructions further causes the computational system to receive the magnetic resonance imaging pulse sequence configuration data in response to inputting the subject data into the graph database. This embodiment may be beneficial because it may provide a means of automatically configuring a magnetic resonance imaging system.

Graph databases, as used herein, encompass databases that use graph structures and encode data and relationship with nodes and edges. The nodes may encode data and the edges may encode relationships between those nodes.

The subject data may be magnetic resonance imaging subject data. The subject data may be data, which is used to describe a subject, describe a magnetic resonance imaging system, provide information on medical questions or suspected health problems with a subject as well as providing measurements or control data from a magnetic resonance imaging system. For example, the subject data may be data measured during the course of a prior magnetic resonance imaging system or previous settings of a magnetic resonance imaging system. In this case the graph database may function as part of a closed control loop for controlling a magnetic resonance imaging system.

In another embodiment the graph database comprises a subject data hypergraph and a pulse sequence configuration hypergraph. The subject data hypergraph comprises subject nodes encoding the subject data. For example, each value of the subject data or type or item of the subject data may be represented by a node in the subject data hypergraph. The subject data hypergraph further comprises subject hyperedges connecting the subject nodes and encoding joint occurrences of the subject data. For example, if certain items of the subject data are present or are joint occurrences, they may be connected together to form relationships. The graph database may for example have a table which lists various subject hyperedges and contains a listing of the various subject nodes that each hyperedge connects.

One point to note is that on a formal level the subject hyperedges may be broken into sub-subject hyperedges. For example, the subject data may contain various types of data, for example, data which is descriptive of the physical condition, size, or medical history of the subject. In other cases, subject data may also comprise data measured using sensors or from previous magnetic resonance imaging scans and grouped together as another group of data. In yet other instances, data descriptive of the type of magnetic resonance imaging system being used and its capabilities and/or accessories or equipment available may comprise another group of data. Each of the sub-groups of data may have their own hyperedges. In this case several hyperedges may be specified for each instance of the subject data. Regardless of if there is a single subject data hypergraph or if this is broken into multiple subject data hypergraphs, the results are formally equivalent.

The pulse sequence configuration hypergraph comprises configuration nodes encoding individual configuration data for an individual magnetic resonance imaging pulse sequence. The pulse sequence configuration hypergraph further comprises configuration hyperedges which connect the configuration nodes to pulse sequence combinations forming the magnetic resonance imaging pulse sequence configuration data. The configuration hyperedges connect together individual magnetic resonance imaging pulse sequences. The configuration hyperedges may then, in some instances, be considered to forming magnetic resonance imaging protocols or a collection of magnetic resonance imaging pulse sequences that are performed for a particular subject.

The database further comprises a main graph data frame listing each subject hyperedge and connections to configuration hyperedges. The main graph data frame listing may for example be a table, which contains this information. When a particular subject hyperedge or a combination of hyperedges, if the hyperedges are formally broken into smaller ones, may be listed and then these links to various configuration hyperedges are provided.

The main graph data frame listing comprises a bridge between the subject data hypergraph(s) and the pulse sequence configuration hypergraph. The subject data may be used to select a particular subject data hyperedge, which then may be mapped onto one or more configuration hyperedges. Each of these hyperedges provides a different magnetic resonance imaging protocol that is comprised from one or more pulse sequences that would be executed by a magnetic resonance imaging system. This system may therefore provide a means of taking the subject data and then automatically providing the configuration data necessary to perform a complete magnetic resonance imaging protocol.

In another embodiment the connections to the configuration hyperedges comprise weighting factors. The graph database is configured to select the magnetic resonance imaging pulse sequence configuration data by using the subject data comprising identifying a closest matching subject hyperedge in the subject data hypergraph using the subject data. The closest matching subject hyperedge in this case may be found using a variety of methods. In one case the subject data may be used to find a subject hyperedge, which contains all of the elements of the subject data. This may be considered then a matching subject hyperedge. However, it may be possible that an identical match to a particular subject hyperedge may not be found. In this case a search could be used to find a closest matching subject hyperedge. For example, the various elements of the subject data may be left out selectively. Once an element or piece or data has been left out from the subject data, a search for a matching subject hyperedge can then be performed again. The closest matching subject hyperedge may for example be the subject hyperedge, which matches with the fewest number of subject data elements that have been left out. In some instances, there may be a listing or ranking or particular order in which various elements of the subject data are left out when the closest matching subject hyperedge is searched for.

The graph database is further configured to select the magnetic resonance imaging pulse sequence configuration data by using the subject data comprising a step of returning configuration hyperedges and weighting data associated with the closest matching subject hyperedge in the main graph data frame. Selection of the magnetic resonance imaging pulse sequence configuration data using the subject data further comprises selecting the magnetic resonance imaging pulse sequence configuration data from the returned configuration hyperedges at least partially using the weighting data.

For example, within the main graph data frame listing for a particular subject hyperedge, there may be links to multiple configuration hyperedges. The weighting data may provide a ranking or rating of each of these configuration hyperedges in the main graph data frame listing. As a simplest means of choosing the closest matching subject hyperedge, the subject hyperedge with the highest weighting factor may simply be selected.

In other examples, other considerations may also be taken into account when choosing the configuration hyperedge. For example, weighting values for the configuration hyperedges don't just have to be on a "this-protocol-for-those-symptoms" level (taken from main graph dataframe, as described above), but also weights could be taken from additional values such as usage counters counters and thumbs-up counters (user ratings) for each individual sequence (node "size" and merits), independent of symptoms, that can be used too when selecting or constructing the magnetic resonance imaging protocol.

In another embodiment each subject data hyperedge is identified by a unique hash number. For example, for each particular classification which might be in the subject data, a hash value might be assigned. This means that particular combinations of values for subject data may then have unique hash numbers. This enables each subject data hyperedge to be identified by a unique hash number or value. Execution of the machine-executable instructions further comprises converting the subject data into a subject hash number and then searching for the closest matching subject hyperedge in the main graph data frame using the subject hash number. For example, the main graph data frame may be a table which lists the various hash values and then in other columns lists matching configuration hyperedges.

If a particular subject hash number does not match an existing subject hyperedge in the main graph data frame, then it may be possible to selectively leave individual elements from the subject data out to see if a match can be obtained by reducing the amount of data being matched. In some instances, there may be a listing so that particular elements of the subject data are selected in a particular order.

In another embodiment the encoding of the magnetic resonance imaging pulse sequence configuration data for an individual pulse sequence is numerical. There are typically various values in a pulse sequence which are numerical. These may for example be various time elements in the pulse sequence as well as various values such as gradient strength or the strength of an RF pulse which is produced. There are also other values which may be considered to be non-numerical. To encode them in a numerical way it is possible to simply assign numerical values to these non-numerical values and provide a mapping which is then numerical. This may have the advantage that various numerical analysis techniques may be applied to the nodes of the pulse sequence configuration hypergraph.

In another embodiment the graph database is configured to add new configuration nodes to the configuration hypergraph by binning the pulse sequence parameters. This may be beneficial because it may provide for a means of grouping functionally equivalent pulse sequences together or by reducing the number of pulse sequences.

In another embodiment the method further comprises calculating a distance within the magnetic resonance imaging pulse sequence configuration data for the configuration nodes connected by the configuration hyperedges associated with the closest subject hyperedge in the main graph data frame. The method further comprises selecting or at least partially combining the magnetic resonance imaging pulse sequence configuration data from the returned configuration hyperedges by using a combination of the weighting data and the calculated distance within the magnetic resonance imaging pulse sequence configuration data.

This embodiment may be beneficial because it may provide for a means of providing for magnetic resonance imaging protocols which are able to provide a more varied result. The distance within the magnetic resonance imaging pulse sequence configuration data may in some instances be considered as a metric. As the configuration nodes have been encoded numerically, it is therefore possible to use these purely numerical configuration data to calculate a distance. As was mentioned before, it may for example be a simple Euclidean distance or it may be a more complicated metric which is able to measure a numerical distance.

In another embodiment the configuration nodes are encoded in a reduced dimensionality state. The memory further comprises a mapping between the reduced dimensionality state and the magnetic resonance imaging pulse sequence configuration parameters. Providing the magnetic resonance imaging pulse sequence configuration data comprises converting between the reduced dimensionality state and the magnetic resonance imaging pulse sequence configuration parameters.

This may be beneficial because not all values within a pulse sequence are independent of each other. The reduced dimensionality state could for example be performed using principle component analysis. This may then reduce the number of variables needed to represent a particular pulse sequence. The calculation of the distance within the magnetic resonance imaging pulse sequence configuration data may have more meaning in this reduced dimensionality state. This may for example help to choose a magnetic resonance imaging protocol, which has a greater diversity of magnetic resonance imaging settings. This may provide a larger variety of magnetic resonance images with the least amount of measurement time.

In another embodiment, execution of the machine-executable instructions further causes the computational system to receive magnetic resonance imaging survey scan data descriptive of the subject. Execution of the machine-executable instructions further causes the computational system to derive at least a portion of the subject data from the survey scan data.

A magnetic resonance imaging survey scan, as used herein, encompasses a magnetic resonance imaging scan, which is performed before other magnetic resonance imaging scans in a magnetic resonance imaging protocol. The magnetic resonance imaging survey scan is typically performed at a lower resolution so that it can be performed quickly and provides an overview of a larger area of the subject. This may for example be used to perform alignment of imaging volumes as well as providing an overview for the clinician or technician to decide what further magnetic resonance imaging scans are to be performed and what the geometry settings of the imaging scan may be (field of view, angulation, shimming). Often times if one were to set up an automated system for configuring and executing magnetic resonance imaging systems it would be beneficial to perform a standardized magnetic resonance imaging survey scan with a large imaging field of view initially. In this embodiment this preliminary or survey scan is then used to provide at least a portion of the subject data.

In another embodiment the deriving of at least a portion of the subject data from the survey scan data comprises determining an image segmentation of the survey scan using an automatic segmentation module. For example, there are a variety of image segmentation modules which are available. For example, a shape-deformable or even segmentation algorithms for specific areas of the body such as locating the position of a diaphragm or using edge detection. The deriving of at least a portion of the subject data from the survey scan data further comprises calculating at least a portion of the subject data using the image segmentation. This may for example be very useful in aligning or configurating the size or orientation of fields of view or imaging volumes in the magnetic resonance imaging system.

In another embodiment the deriving of at least a portion of the subject data from the survey scan further comprises calculating at least a portion of the configuration data using the image segmentation and the pre-scan or survey data. The derivation of at least a portion of the subject data from the survey scan data further comprises giving the operator a suggestion on how to adjust the imaging configuration settings for better expected image quality. For example, this may provide settings or suggested settings or list of various protocols that may be selected by the operator.

In another embodiment the memory further comprises an image classification machine learning module. The deriving of at least a portion of the subject data comprises receiving an image classification in response to inputting the survey image data into the image classification machine learning module and then providing the image classification as the at least one portion of the subject data.

For example, the image classification machine learning module could be implemented as a convolutional neural network, a random forest, or a support vector machine. These are basically various techniques which may be used to receive an image or three-dimensional data and output a classification. For example, if a convolutional neural network is used a variety of labeled survey scans with various classifications may be used for training it. The various types of neural network architectures could be used for implementing the image classification learning module. In some instances, a U-net may be used, a ResNet, or other types of convolutional neural networks typically used for performing classification on images.

In another embodiment deriving of at least a portion of the subject and/or configuration data further comprises determining if signal quality is sufficient for the planned acquisition and whether sequence settings may possibly be adjusted. This for example may be performed using the image classification learning module also. The survey scan could be input into the image classification learning module and it may output one or more signal values, such as the signal-to-noise or image contrast and this may be used to provide an estimate of what the signal quality might be. This might be useful in adjusting the pulse sequence parameters before they are performed.

In another embodiment, sequence configuration parameters or instances for which the suggestion algorithm had higher uncertainty (e.g., sensitive fat suppression settings or very abnormal geometries) could be presented to the operator in a highlighted way. In a non-expert user interface mode, sequence parameters not advised to be changed or determined with high fidelity could be hidden from the user, revealing a smaller set of "modifiable" parameters to facilitate an easier magnetic resonance imaging user interface. These modifiable parameters do not have to be classical user interface settings as found on magnetic resonance scanners today, but they can also be surrogate contrast control parameters, defined using functional mapping, dimensionality reduction, or machine learning techniques. This could offer a simplified user experience and enable "non-expert" operators to easily tune typical parameter combinations to alleviate certain observed image quality problems.

In another embodiment, execution of the machine-executable instructions further causes the computational system to receive a selection of the magnetic resonance imaging system. Execution of the machine-executable instructions further causes the computational system to translate the received magnetic resonance imaging pulse sequence configuration data to one or more pulse sequences configured to control the magnetic resonance imaging system to acquire k-space data of the subject. This embodiment may be beneficial because it may provide a device agnostic means of providing magnetic resonance imaging protocols to magnetic resonance imaging systems and enabling one database to host information for all scanners, without the need for separate hardware- or vendor-specific databases.

For example, if the magnetic resonance imaging pulse sequence configuration data has been encoded numerically this translation may involve a step of transforming the numerical codes back into pulse sequence settings. The numerical encoding for example may enable a translation from a generic or purely numerical representation into device or magnetic resonance imaging-specific pulse sequence commands and protocols.

In another embodiment, the medical system further comprises one or more magnetic resonance imaging systems. Execution of the machine-executable instructions further causes the computational system to acquire the k-space data of the subject by controlling the one or more magnetic resonance imaging systems with the one or more pulse sequences. In most cases, the medical system would comprise the computational system and graph database that is in communication with the one or more magnetic resonance imaging systems. For a particular subject located at a particular magnetic resonance imaging system the subject data would be sent and then in response, the one or more pulse sequences would be provided to perform a particular magnetic resonance imaging protocol. This embodiment may be beneficial because it may provide for a means of automating or semi-automating the acquisition of k-space data from subjects at one or more locations.

In another embodiment, execution of the machine-executable instructions further causes the computational system to reconstruct one or more magnetic resonance images from the k-space data. This for example could be performed by the magnetic resonance imaging system that acquired the k-space data. In other instances, this k-space data could be sent to a cloud-based or internet-based service for reconstructing the images. This service may or may not be located at the same location as the graph database.

In another embodiment, when a magnetic resonance image is acquired, this is then processed for example using the segmentation module and/or pattern recognition module and this is then used to generate further subject data, which may be used to select further magnetic resonance imaging protocols or modify one that has been partially performed. This may result in a closed control loop while operating a magnetic resonance imaging system in conjunction with the use of the graph database.

In another aspect, the invention provides for a method of operating a medical system. The medical system comprises a computational system. The medical system further comprises a memory storing machine-executable instructions and a graph database. The graph database is configured to output magnetic resonance imaging pulse sequence configuration data in response to receiving subject data. The method comprises the computational system receiving the subject data. The method further comprises receiving the magnetic resonance imaging pulse sequence configuration data in response to inputting the subject data into the graph database. Inputting the subject data into the graph database may also be interpreted as querying the graph database with the subject data.

In another aspect the invention provides for a computer program comprising machine-executable instructions for execution by a computational system. Execution of the machine-executable instructions causes the computational system to receive the subject data. Execution of the machine-executable instructions further causes the computational system to receive the magnetic resonance imaging pulse sequence configuration data in response to inputting the subject data into a graph database. The graph database is configured to output magnetic resonance imaging pulse sequence configuration data in response to receiving the subject data.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A `computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

`Computer memory' or 'memory' is an example of a computer-readable storage medium. Computer memory is any memory which is directly accessible to a computational system. `Computer storage' or 'storage' is a further example of a computer-readable storage medium. Computer storage is any non-volatile computer-readable storage medium. In some embodiments computer storage may also be computer memory or vice versa.

A `computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A `user interface' as used herein is an interface which allows a user or operator to interact with a computer or computer system. A `user interface' may also be referred to as a `human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A `hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include,but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or `display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

K-space data is defined herein as being the recorded measurements of radio frequency signals emitted by atomic spins using the antenna of a Magnetic resonance apparatus during a magnetic resonance imaging scan.

A Magnetic Resonance Imaging (MRI) image or MR image is defined herein as being the reconstructed two- or three-dimensional visualization of anatomic data contained within the magnetic resonance imaging data. This visualization can be performed using a computer.

### BRIEF DESCRIPTION OF THE DRAWINGS

In the following preferred embodiments of the invention will be described, by way of example only, and with reference to the drawings in which:
Fig. 1 illustrates an example of a medical system;
Fig. 2 shows a flow chart which illustrates a method of using the medical system of Fig. 1;
Fig. 3 illustrates a further example of a medical system;
Fig. 4 shows a flow chart which illustrates a method of using the medical system of Fig. 3;
Fig. 5 illustrates a further example of a medical system;
Fig. 6 exemplary graph database displayed in tabular form;
Fig. 7 illustrates several examples of a magnetic resonance imaging scan setting table which holds the node information for the configurations hypergraph; and
Fig. 8 graphical representation of a graph database.

### DETAILED DESCRIPTION OF EMBODIMENTS

Like numbered elements in these figures are either equivalent elements or perform the same function. Elements which have been discussed previously will not necessarily be discussed in later figures if the function is equivalent.

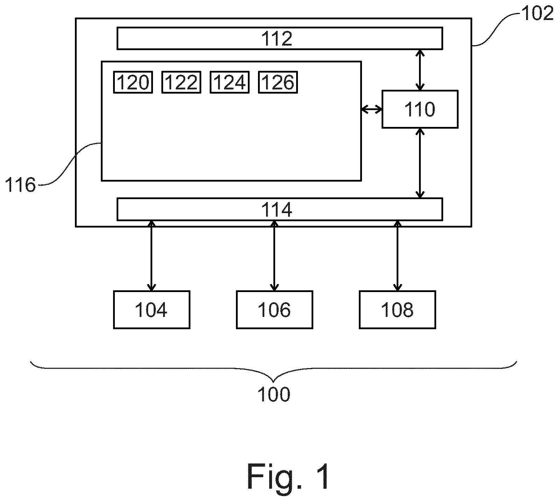

Fig. 1 illustrates an example of a medical system 100. The medical system 100 is shown as comprising a computer 102 and optionally, a magnetic resonance imaging system 104, an optional magnetic resonance imaging system 106, and an optional magnetic resonance imaging system 108. The optional magnetic resonance imaging systems 104, 106, and 108 are intended to illustrate that the medical system 100 could form the core of a system which integrates multiple magnetic resonance imaging systems 104, 106, 108.

The computer 102 is shown as comprising a computational system 110. The computer 102 is intended to represent one or more computers that may be located at one or more locations. Likewise, the computational system 110 could represent multiple computational systems or processors or cores located at one or more locations also.

The computational system 110 is shown as being connected to an optional user interface 112. The user interface 112 may enable an operator or a controller to control and interact with the medical system 100. The computational system 110 is further shown as being connected to a network interface or hardware interface 114. The network interface may be used to communicate with other devices or components of the medical system 100. For example, the network interface 114 is shown as forming network connections with the magnetic resonance imaging systems 104, 106, and 108.

The computational system 110 is further shown as being in communication with a memory 116. The memory 116 is intended to represent various types of memory which are accessible to the computational system 110. In one example, the memory 116 may be a non-transitory storage medium.

The memory 116 is shown as containing machine-executable instructions 120. The machine-executable instructions 120 may comprise instructions which enable the computational system 110 to perform basic image and numerical tasks. In some examples the machine-executable instructions 120 may enable the computational system 110 to control other components of the medical system 100 such as the magnetic resonance imaging systems 104, 106, and 108. The memory 116 is further shown as containing a graph database 122. The memory 116 is further shown as containing subject data 124 which may be used as input or to query the graph database 122. The memory 116 is further shown as containing the magnetic resonance imaging pulse sequence configuration data 126.

The computer 102 could for example be a remote server or cloud-based system that hosts or provides access to the graph database 122.

The magnetic resonance imaging pulse sequence configuration data 126 was received in response to querying the graph database 122 with the subject data 124. In some examples the magnetic resonance imaging pulse sequence configuration data 126 is in the form of multiple pulse sequences, which may be used to control one or more of the magnetic resonance imaging systems 104, 106, and 108. In other examples the magnetic resonance imaging pulse sequence configuration data 126 would have to go through a conversion process to convert it into one or more pulse sequences that would then be used to control the magnetic resonance imaging systems 104, 106, and/or 108.

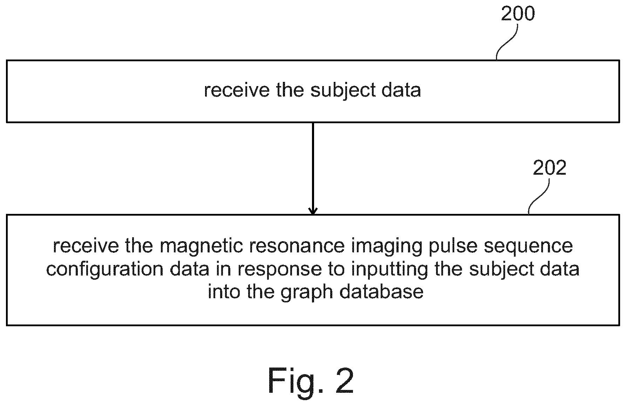

Fig. 2 shows a flowchart which illustrates a method of operating the medical system 100 of Fig. 1. In step 200, the subject data is received. In step 202, the magnetic resonance imaging pulse sequence configuration data 126 is received in response to inputting or querying the graph database 122 with the subject data 124.

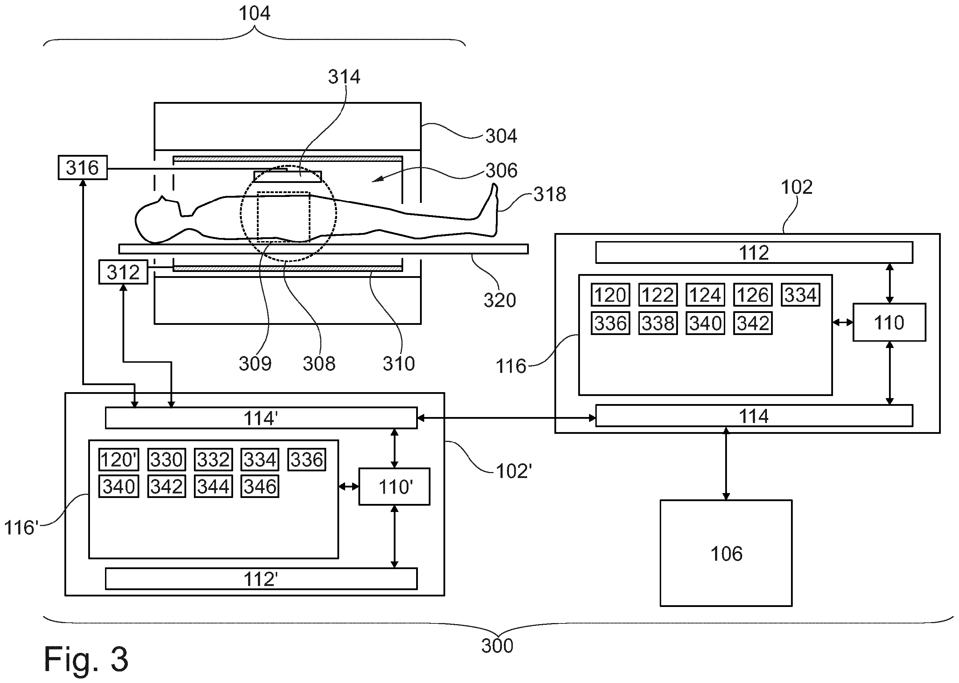

Fig. 3 illustrates a further example of a medical system 300. This is shown as comprising the computer 102 of Fig. 1 as well as the magnetic resonance imaging system 104 and 106. The components of the magnetic resonance imaging system 104 are shown in detail.

The magnetic resonance imaging system 104 comprises a magnet 304. The magnet 304 is a superconducting cylindrical type magnet with a bore 306 through it. It is also possible to use both a split cylindrical magnet and a so-called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject. The arrangement of the two sections area is similar to that of a Helmholtz coil. Open magnets are popular because the subject is less confined. Inside the cryostat of the cylindrical magnet there is a collection of superconducting coils.

Within the bore 306 of the cylindrical magnet 304 there is an imaging zone 308 where the magnetic field is strong and uniform enough to perform magnetic resonance imaging. A field of view 309 is shown within the imaging zone 308. The k-space data are acquired for the field of view 309. The region of interest could be identical with the field of view 309 or it could be a sub volume of the field of view 309. A subject 318 is shown as being supported by a subject support 320 such that at least a portion of the subject 318 is within the imaging zone 308 and the field of view 309.

Within the bore 306 of the magnet there is also a set of magnetic field gradient coils 310 which is used for the acquisition of measured k-space data to spatially encode magnetic spins within the imaging zone 308 of the magnet 304. The magnetic field gradient coils 310 are connected to a magnetic field gradient coil power supply 312. The magnetic field gradient coils 310 are intended to be representative. Typically, magnetic field gradient coils 310 contain three separate sets of coils for spatial encoding in three orthogonal spatial directions. A magnetic field gradient power supply supplies current to the magnetic field gradient coils. The current supplied to the magnetic field gradient coils 310 is controlled as a function of time and may be ramped or pulsed.

Adjacent to the imaging zone 308 is a radio frequency coil 314 for manipulating the orientations of magnetic spins within the imaging zone 308 and for receiving radio transmissions from spins also within the imaging zone 308. The radio frequency antenna may contain multiple coil elements. The radio frequency antenna may also be referred to as a channel or antenna. The radio frequency coil 314 is connected to a radio frequency transceiver 316. The radio frequency coil 314 and radio frequency transceiver 316 may be replaced by separate transmit and receive coils and a separate transmitter and receiver. It is understood that the radio frequency coil 314 and the radio frequency transceiver 316 are representative. The radio frequency coil 314 is intended to also represent a dedicated transmit antenna and a dedicated receive antenna. Likewise, the transceiver 316 may also represent a separate transmitter and receiver. The radio frequency coil 314 may also have multiple receive/transmit elements and the radio frequency transceiver 316 may have multiple receive/transmit channels.

The magnetic resonance imaging system 104 is shown as comprising a computer 102' that also comprises a computational system 110', a hardware interface 114', an optional user interface 112', and a memory 116'. In other examples, the features and components of the computer 102' and 102 could be combined. Instead of having a central computer 102 serving several magnetic resonance imaging systems 104, 106, as is depicted in Fig. 3, the functionality of the computer system 102 could be integrated into the computer 102' that controls the magnetic resonance imaging system 104 locally.

The memory 116' of the computer 102' is shown as comprising machine-executable instructions 120'. The machine-executable instructions 120' contain instructions which may enable the computational system 110' to perform such tasks as image reconstruction, data manipulation, and the control of the other components of the magnetic resonance imaging system 104.

The memory 116' is shown as containing survey scan pulse sequence commands 330. In an automated system the survey scan pulse sequence commands 330 could for example be pulse sequence commands that are executed automatically when a subject 318 is inserted into the magnetic resonance imaging system 304 and reaches the imaging zone 308. The memory 116' is further shown as containing survey scan k-space data 332. This is the acquired k-space data that is acquired by controlling the magnetic resonance imaging system 104 with the survey scan pulse sequence commands 330. The memory 116' is further shown as containing survey scan data 334, which could be a survey scan image that is reconstructed from the survey scan k-space data 332. The memory 116' is further shown as containing local subject data 336. This could contain such things as a selection of a region of the subject 318 to be imaged, details about the subject 318 such as the weight, age, or other metadata descriptive of the subject 318.

The memory 116 in the computer 102 is shown as also containing the survey scan data 334 and the local subject data 336. The local subject data 336 could also contain information that is descriptive of the particular magnetic resonance imaging system 104 and its capabilities, such as what hardware is available or not available.

The memory 116 is further shown as containing an image classification machine learning module 338. The memory 116 is further shown as containing an image classification 340 that is received from the image classification machine learning module 338 in response to receiving the survey scan data 334 as input. For example, there could be a standard convolutional neural network that is used to classify images. The image classification 340 may for example be concatenated with the local subject data 336 to produce the subject data 124. This may then be input into the graph database 122 to produce the magnetic resonance imaging pulse sequence configuration data 126. The magnetic resonance imaging pulse sequence configuration data 126 may then be converted into one or more pulse sequences 342 specifically tailored or modified for the magnetic resonance imaging system 104.

It can also be seen that the one or more pulse sequences 342 have been transferred to the memory 116. The memory 116' is further shown as containing k-space data 344 that has been acquired by controlling the magnetic resonance imaging system 104 with the one or more pulse sequences 342. The memory 116' is further shown as containing one or more magnetic resonance images 346 that have been reconstructed from the k-space data 344.

In other examples, the one or more magnetic resonance images 346 could for example be further processed by the image classification machine learning module 338 or a similar module and used to further refine or control the magnetic resonance imaging system 104 for further examinations of the subject 318. In this way, a closed control loop may be formed.

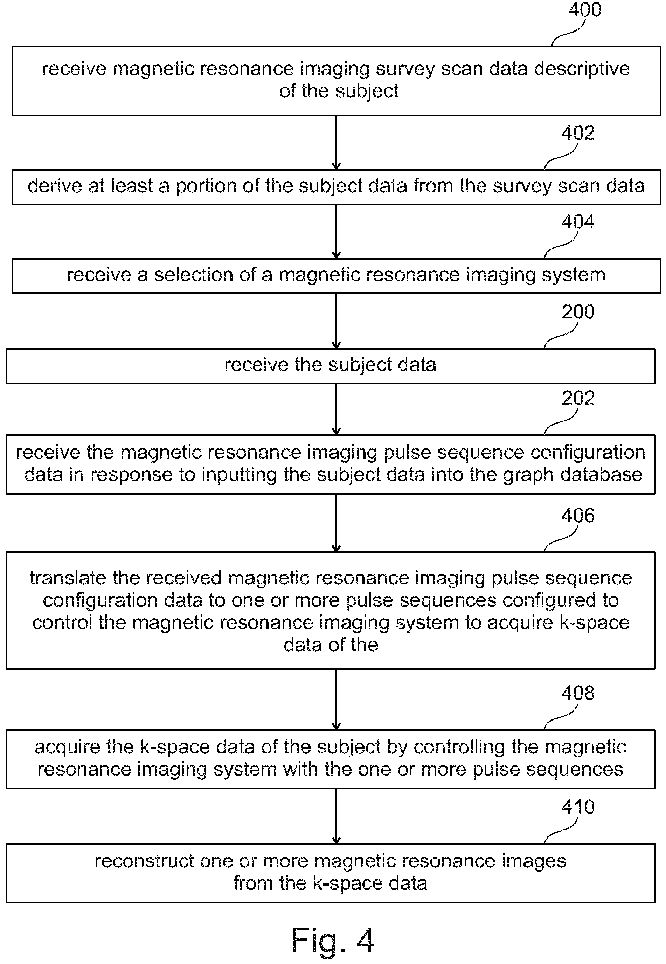

Fig. 4 shows a flowchart which illustrates a method of operating the medical system 300 of Fig. 3. In step 400 the magnetic resonance imaging survey scan data 334 is received. In step 402, at least a portion of the subject data 324 is derived from the survey scan data 334. In step 404, a selection of the magnetic resonance imaging system 104 is received. Steps 200 and 202 are performed as they were illustrated in Fig. 2. In step 406 the magnetic resonance imaging pulse sequence configuration data 326 is translated into one or more pulse sequences 342; they are configured to control the magnetic resonance imaging system 104 to acquire the k-space data 344. In step 408, the k-space data 344 is acquired by controlling the magnetic resonance imaging system 104 with the one or more pulse sequences 342. Then, in step 410, the one or more magnetic resonance images 346 are reconstructed from the k-space data 344.

Examples may provide for an MRI protocoling automation tool and underlying system. The backend includes a graph database (122) which enables a scalable and well-maintainable way to automate MRI protocoling based on patient indications and other variables or data. The proposed system may generalize well across different scanners, sites and vendors, potentially solving many open issues in MRI protocoling automation. The envisioned database connects subject data (124) leading to an MRI order (magnetic resonance imaging pulse sequence configuration data 126) with appropriate MRI protocols and possibly other related patient information. The database can constantly grow during use and be used for MRI protocoling inference, suggesting the best scan combinations for new patients. As a graph database, it emphasizes connections and mutual occurrence of different elements which may provide for a means of determining MRI exams for different clinical questions. This is a crucial component for the future autonomous MRI scanner, operating without local trained personnel, and it is expected to gradually standardize the MRI sequences used across different clinics and practices on the way there. This invention does not only offer new functionality to MRI operators, but also creates many possibilities for the maintaining vendor by automatically collecting and structuring detailed information about the MRI scanner usage in different fields, filterable by symptoms and body parts. Applicable dimensionality reduction techniques could offer a new view on MRI sequence parametrization and enable new approaches to sequence and protocol development.

Currently, radiologists prescribe a specific MRI scan set (exam/protocol) with desired contrasts based on the patient's complaints and the referring physician's conjectures of possible underlying causes. The contrasts of following MRI scans are based on radiologist/technologist training and a limited set of experience from several people. Their starting point is usually a local database of preconfigured scan cards (vendor pre-sets), which are chosen based on patient indications and possibly fine-tuned by the on-site staff. The local databases are often not left factory set, but instead preconfigured by local radiology staff. All this leads to large variability in the use of MRI at different sites because every team has its own experience and distinct preferences for sequences and settings, resulting in often hardly comparable contrasts of scans from different sites.

State-of-the art MRI scanners do not systematically collect structured information about the detailed scanner usage at clinical sites. Usage statistics can only be extracted from general local data logging which is only done for maintenance purposes.

The herein described architecture may enable the growth of a graph database, capturing complex relationships between patient symptoms and typically prescribed MRI scans in enough detail to use this database to suggest suitable MRI protocols for patients with similar indications. Solving this with, in some examples a machine learning solution is suboptimal because it is not comprehensible or transparent in its decision support, hard to curate and systematically correct for errors or updates to gold-standards or regulations (needs full retraining, excluding the corresponding scans), it can hardly be monitored, and it is not clear when an input of patient indications cannot be sensibly processed because there is no or too little training data for those input symptoms, among other problems of scalability, maintainability, and accountability.

A difficulty in the collection and storage of relevant data lies in the different layers of connectivity that are of interest:
- connectivity of different symptoms/indications (from co-occurrence),
- connectivity of different MRI sequences (from combination in a protocol), and
- connectivity between symptom combinations and MRI scans/protocols (exam design for a patient).

A joint occurrence of certain symptoms needs to be recorded and searchable as such, since a similar combination of symptoms or a subset thereof may lead to entirely different suspicions of the underlying causes, calling for different scans. Therefore, the connections between different symptoms or subject data are of interest, but also the connections of individual symptom conglomerates to MRIs (and not just individual scans, but scan combinations, i.e., connections between MRI sequences are important for sensible protocol construction with minimal redundancy of acquired contrasts). Some examples may handle this by recording all these connectivity layers in an easily searchable and extendible format in the graph database, preserving the freedom to apply different filtering and processing methods or AI algorithms during prediction usage of the MRI protocol advisor tool, allowing to constantly improve the automated scan suggestion system. This approach also shifts the machine learning input solely to the MRI sequence/protocol domain, which is better defined, more structured and limited than the indications/symptoms word cloud domain. The generation and curation of training data may be less problematic in our case, as it could be extracted from existing log files retrospectively to find out which sequences are popularly used together in protocols. Synthetic training data generation could be applied as well, based on simulated contrast similarity.

Examples may provide one or more of the following benefits:
- less clinical staff time spent on picking and tuning exams, more of an approving function, which could be provided by remote operation,
- building intrinsic expert knowledge databases for the application of MRI scanners in the clinics,
- better diagnostic outcome and protocol acceleration by highly optimized MRI sequences and combinations thereof for different body parts and disease classes, based on the collective radiology experience,
- and more standardized MRI exams being prescribed, less imaging variability and better comparability.

Examples may provide for a software tool for mapping medical symptoms/indications of a patient to a suitable set of MRI scans for that patient, with the ambition of yielding a time-efficient MRI protocol with high likelihood of answering the clinical question. The main application is an automated MRI protocoling tool. A differentiating feature is its graph database and its use during protocol inference, as opposed to trained machine learning models linking indications and useful MRI sequences end-to-end. The underlying system of interconnected sub-databases ("graph database") is the backbone of this MRI protocoling tool.

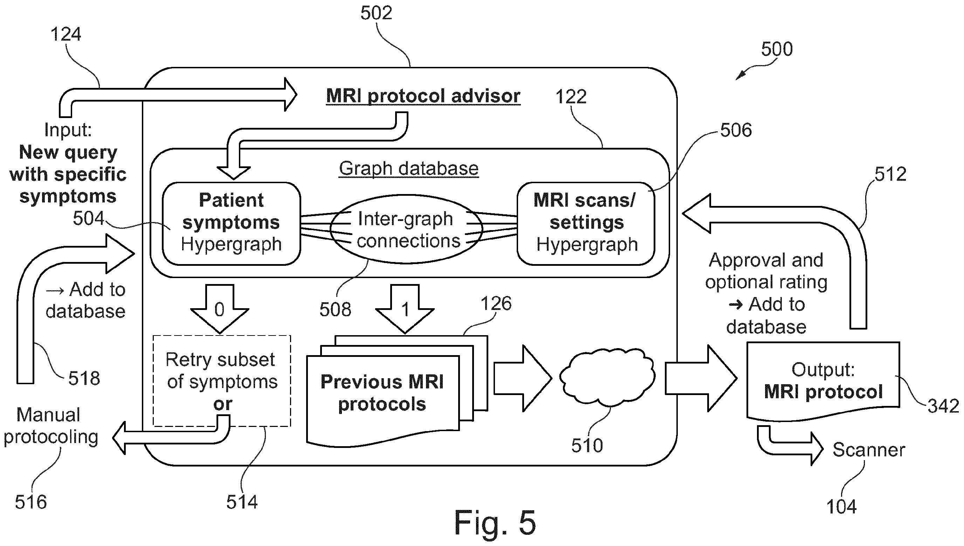

Fig. 5 illustrates a further example of the medical system 500. In 500 a functional illustration of a magnetic resonance imaging protocol adviser 502 is illustrated. As input, the MRI protocol adviser 502 takes the subject data 124. In this example there is a new query with specific symptoms that are detailed. However, the symptoms are used purely as an example and machine configure and the data measured from the subject may also be used in addition or instead. The MRI protocol adviser 502 comprises the graph database 122; it comprises a subject hypergraph and a pulse sequence configuration hypergraph 506 that are connected by a main graph data frame 504 that provides a mapping between the subject hyperedges of the subject hypergraph 504 with configuration hyperedges in the pulse sequence configuration hypergraph 506. The subject data 124 is used to query the graph database 122 via the subject hypergraph 504. When a matching subject hypergraph is found the main graph data frame 508 is used to find multiple magnetic resonance imaging pulse sequence configuration data 126.

There may for example be weighting factors or other data which is also included to rate the quality of the different MR protocols and individual sequences. There is an decision module 510 which may be for example implemented as a logical decision module or using an artificial intelligence module such as a neural network or decision tree, which selects particular sequences from the protocol(s) 126 and then the system outputs the one or more magnetic pulse sequences 342. These may then be provided to the magnetic resonance imaging scanner 104 to acquire k-space data. In some examples, the radiologist may be able to rate the quality of the images or machine learning classifiers may rate the images for quality and artefacts and these are optionally provided via an approval feedback mechanism 512a to update the weighting factors of the relevant hyperedges in the pulse sequence configuration hypergraph 506. This is done by modifying the main graph data frame 508 and the respective imaging sequence configuration nodes' attributes (e.g., usage frequency and popularity in diagnosis from one-click feedback) in the configuration hypergraph 506.

In some instances, the subject data 124 may not provide for a perfect match with a subject hyperedge in the subject hypergraph 504. Box 514 represents retrying a search for a matching subject hyperedge by trying a subset of the subject data. If this does not find something, in some examples the system may then go to a manual protocoling tool, which enables the operator to manually select or construct the magnetic resonance imaging protocol to use. This may for example also be added to the database 518 to improve the amount of information in the graph database 122.

The MRI protocol advisor (502) has access to the graph database (122), which contains information about co-occurring symptoms and corresponding MRI protocols conducted in previous cases. It can be described using hypergraphs, where an edge can connect more than two vertices. Each hyper-edge in the two separate subgraphs "patient symptoms" (or subject data 504) and "MRI scans/settings" (pulse sequence configuration hypergraph 506) which represent a previous case by connecting individual elements (symptoms/MRI sequences) in each subgraph. The hyper-edges in the two subgraphs may get weighted inter-graph connections in the database to link specific indications with MRI sequence combinations.

The MRI protocol advisor tool (502) of Fig. 5 queries the graph database by searching for the symptoms combination of a new patient to find previously conducted MRI protocols for those symptoms. If the queried symptoms were not found in the database (case 0), it can be searched with subsets of the original symptoms input. If this fails or is not desired, manual protocol planning is necessary. The classically planned MRI exam is then added to the database with the corresponding symptoms combination. If the database search was initially successful (case 1), it yields all previously conducted MRI protocols for those indications, including detailed sequence settings. To compose the best suited MRI protocol for the new patient, AI can be used to minimize redundancy in contrasts and optimize protocol length, possibly with some additional patient information as input (e.g., implants). Previously used protocols can me mixed and specific sequences can be excluded based on compatibility with the site and patient, due to a clear and comprehensive parametrization of sequence settings in the database. Once approved, the protocol can be used on the scanner and added to the database.

Some examples may comprise one or more of the following features:
- The graph database to store structured information about nodes in different subgraphs (individual symptoms/MRI sequences), hyper-edges within each subgraph (full indications/MRI protocols), and connections between the hyper-edges of different subgraphs (which MRI for what?). This can be implemented with three separate data tables (details in next section):
   1. An indexed list of patient symptoms/indications,
   2. A table with index combinations from the first table and connected combinations of indices into the third table, and
   3. A third, indexed table with columns corresponding to the settings of a real MRI scan.
- A hardware- and software-specific transformation function to map scanner-specific settings to a generalized space of MRI settings, ideally for each scanner release. This generalized scan settings space composes the columns of the third table mentioned just above. The transformation function may possibly have an inverse to map general MRI settings to scanner-specific sequence specifications.
- An algorithm for the automated addition of new data to the database (described in the next section).
- The MRI protocol advisor tool, consisting of:
- An algorithm for automated searches of the database using patient indications and the table indices, extraction of corresponding information for a given index (previously used MRI protocols).
- A (possibly AI-backed) software framework that takes a list of scan settings from previously conducted MRI exams (output of the component just above) and suggests a sensible MRI protocol for a new patient, with the aim of maximizing diagnostic value while minimizing the protocol duration.

### A) Database construction:

The envisioned graph database consists of several tables. They are called A. 1 - A.3 in this example.

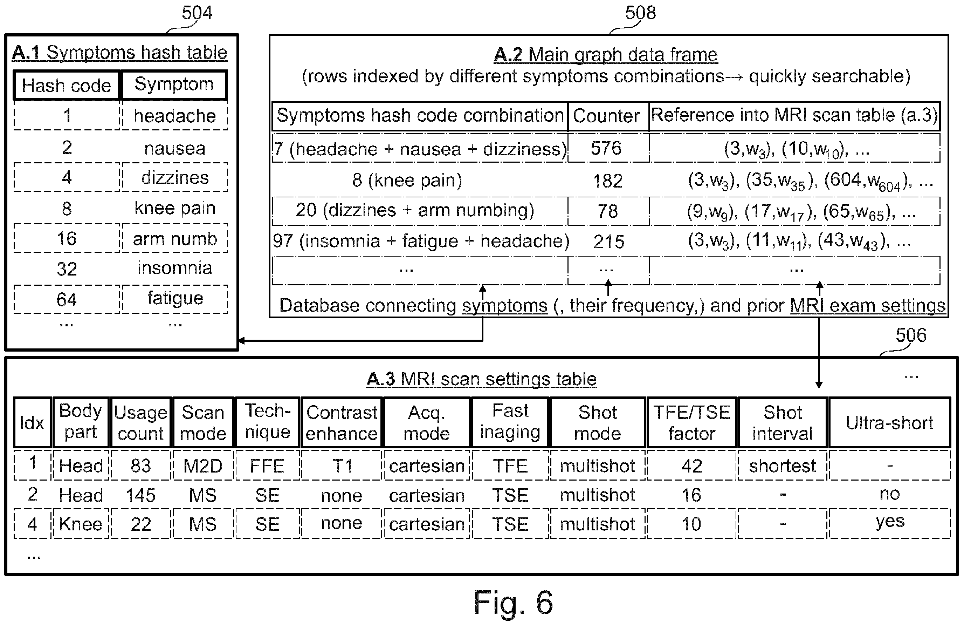

Fig. 6 illustrates a further example of a graph database 122 laid out in table form. There is a symptoms hash table (A. 1) 504, which is equivalent to the subject hypergraph node information. There is a magnetic resonance imaging scan setting table (A.3), which is equivalent to the pulse sequence configuration hypergraph node information 506. Finally, there is a main graph data frame (A.2) 508, which provides information about combinations of subject nodes from (A. 1) 504 and the pulse sequence configuration nodes in (A.3) 506. The hash codes in (A.2) 508 are shown with specific references into the patient symptoms table (A.1) 504 and the MRI scan table (A.3) 506.

Describing this in greater detail, Fig. 6 shows a schematic example of an implementation of the graph database in the most basic embodiment. The main graph data frame (A.2) 508 connects the elements of the independent tables (A. 1) 504 and (A.3) 506 among themselves, but also with each other. Individual symptoms are hashed in (A. 1) 504 and MRI scan settings for specific, individual scans are hashed in (A.3) 506 using the index (idx). Table (A.2) 508 connects symptomatic combinations with scan combinations conducted priorly by recording corresponding hash code combinations in rows. Scan combinations conducted for certain symptoms are weighted (illustrated by the tuple of a hash reference x and a weight wx). The weights can be multi-layered, e.g., frequency-based and "diagnostic quality"-based, and the references may include more attributes. Note: for illustration purposes, (A.3) 506 is presented in clear text. A numerical and compressible version of (A.3) 506 is discussed below in subsection (A.3) 506.
A. 1. Symptoms hash table - A list of hash codes and associated medical conditions/symptoms, e.g., like:

| HASH CODE INDEX | CONDITION |
|---|---|
| 1 | headache |
| 2 | nausea |
| 4 | dizziness |
| 8 | knee pain |
| 16 | arm numbing |

The elements for this hash list can be extracted from the physician's prescribing tag (e.g., the ICD-10 code or indications noted), or the list of all available ICD-10 codes could be used. One may be able to scan the prescribing physician's report with an AI for NLP (natural language processing) to extract more comprehensive information about the patient from free text. New terms deemed as relevant by the NLP-AI could be added to the hash list upon approval by a data curator or manually.
A.2. Main graph data frame - A table recording the mutual occurrence of symptoms and associated MRI scans conducted. This is the main data frame which holds the graph edge information, i.e., connections between patient symptoms (A.1) 504 and MRI scan parameters (A.3) 506.

When a new patient undergoes an MRI exam and is to be added to the database, the symptoms leading to the examination are translated to their hash codes using the lookup table (A. 1) 504. The attained hash codes from (A. 1) 504 are summed up to get a "total" hash code containing all the present symptoms (this can unambiguously be decomposed into the individual symptoms again). The total hash code is used as an index into the main graph data frame (A.2) 508, making it easily searchable and amendable when growing and using the database. Each such hash code combination represents a hyper-edge in the symptoms subgraph, with the elements of (A. 1) 504 as nodes.

Under a certain index (symptoms combination), this data frame (A.2) 508 holds weighted references to MRI exams (scan combinations) conducted before for these symptoms. Essentially, table (A.2) 508 holds connections between the hyper-edges of different subgraphs; the subgraph hyper-edges are stored through the index-combinations from (A.1) 504, (A.3) 506 and possibly others, while inter-graph edges arise from connections between index combinations from the sub-tables complementary to (A.2) 504 in the rows of (A.2) 508.

A new patient (anonymized; only symptoms and conducted scans are of interest in this embodiment) is added to the database at scan time in the following, automated way. The symptoms (extracted from the prescribing physician's note) are converted to a hash code combination and the index of (A.2) 508 is searched for this hash code. If the combination is not present yet, the operators need to manually plan the exam, as is currently standard practice. A new row is added to the data frame (A.2) 508 with information about the MRI scans conducted for the new symptoms combination. This can be realized through weighted references to a third database (A.3) 506, which holds the detailed settings of individual MRI sequences used before. If the symptoms hash combination was already present in (A.2) 508, the protocoling advisor can suggest a protocol, which can be accepted, edited, or rejected by staff. Successfully conducted MRI scans are added in the corresponding row of (A.2) 508 and an occurrence counter is incremented for that index, generally recording the prevalence of different symptomatic combinations and MRI sequences used.

A new MRI exam reference is added into a row of (A.2) 508 as follows (cf. Fig. 6):
For each individual scan in the new patient's MRI examination, check in (A.3) 506 if it already exists there with those settings. If yes, note the (A.3)-index 506 to be summed with the corresponding indices of the other scans in the exam. If a scan does not exist in (A.3) 506 yet, it is queued to be added to (A.3) 506 under a new index, which is used for the hash code sum. The "total exam" hash code sum (hyper-edge on (A.3)-elements 506) is used as a reference and recorded in (A.2) 508 (see third column of table (A.2) in Fig. 6) in the corresponding symptoms combination row. If the reference already exists in the same row of (A.2) 508, i.e., such a protocol has been used for these symptoms before, then the weight *wₓ* of the exam reference x in that (A.2)-row 508 is incremented. In table (A.3) 506, the usage counter (own column) at each scan's index is incremented to record how many times it has been used in total and optional one-click feedback given by the radiology staff on an image that turned out great or very badly can be recorded in the same way.

The graph data frame (A.2) 508 can hold more information about the connections of the databases (A.1) 504 and (A.3) 506. For example, the references into (A.3) 506 could have frequency and quality weights, indicating how often a certain scan combination was performed for a certain symptoms combination (frequency), but also the chance of a scan leading to a diagnosis, separately (diagnostic value/quality). The quality rating could be obtained by implementing a simple one-click feedback option into the viewer used by radiologists. E.g., there could be a "thumbs up" icon in a corner of the displayed image. Optionally, a radiologist could give the one-click feedback if a scan enabled a diagnosis. This positive rating could be collected in table (A.3) 506 in its own column for general, scan-wise diagnostic value statistics, but also in the references of table (A.2) 508 for specific indications. Over time, this quality rating may possibly have more impact on the suggested scans than the frequency weight (the prioritization can be continuously adjusted in the interchangeable AI or classical algorithms of the MRI protocol advisor, described in more detail below in subsection B). For the implementation of such weights, the references in the third column of table (A.2) 508 into (A.3) 506 could be more general data structures, with a combined index and its corresponding weights/attributes (represented by tuples in Fig. 6).

### A.3. MRI scan settings table 506 - A table to contain the settings of all MRI scans conducted.

Some prior notes: The design of this data structure may possibly allow for correct coarse-graining of the information about different MRI scans, i.e., recognizing when two scans are similar enough to be recorded as essentially the same scan. For this, we can define two distinct groups of scan settings: we call them hard parameters and soft parameters here. Hard MRI settings are parameters with categorical or discrete character, where only a few distinct values are possible and define the sequence character (e.g., scan technique, fat suppression, etc.). Soft parameters are quantitative settings that have a rather continuous domain (e.g., echo times, gradient bandwidth, etc.) and tune the contrast or acquisition settings gradually. Each available scan setting of an MRI may possibly be classified manually as a hard or soft parameter.

To generalize the data representation over different MRI hardware and software releases, a generalized MRI scan settings space may possibly be defined, which treats every possible settings parameter as an individual numeric dimension. Each column of table (A.3) 506 is a dimension of this "Generalized Scan Settings Space" (G3S). For each MRI scanner and software release, a function needs to be defined to map the actual scan settings, adjustable in the scanner UI, to the G3S representation (giving all settings a numerical value) and this function may possibly be invertible for each system (to later translate a scan from the G3S to a specific scanner). We call this function the "Hardware Transform Function" (HTF) here. It facilitates one shared database for all scanners and releases. Such functions may possibly in principle be devisable for scanners from other vendors as well.

The index of table (A.3) 506 may possibly allow for unambiguous combinations, like table (A.1)'s 504 hash code index. This is because a combination of different MRI scans in one exam may possibly be identified in the graph database (A.2) 508 as one entity since combined scans are often complementary to each other and required to answer the clinical question. Each individual new scan is recorded in table (A.3) 506 with a new index, but the references made in (A.2) 508 are scan combinations (cf. third column of (A.2) 508 in Fig. 6).

The large number of columns of (A.3) 506 may possibly make it computationally demanding to add new MRI scans to the database (A.3) 506 at some point, since all column values (settings) have to be compared to find a match and use an existing index for reference. This is only relevant when adding a new patient to the database, which does not have to be fast and could be done in the cloud or on own servers with a queue system. The computational and data storage load can also be alleviated by a dimensionality reduction, which offers other advantages as well (details below).

At an early stage, the representation of an MRI scan in this database (A.3) 506 may possibly be quite detailed and consist of all settings translated from the scanner UI by the HTF (from all parameters possibly listed under all tabs: contrast, acceleration, etc.). The body part(s) scanned are an important parameter, recorded in a categorical column to make the scan database (A.3) 506 easily filterable by body parts. Hard parameters already allow for only a finite amount of discrete values. The soft parameter domains may possibly be defined as histogram bins with sensible bin widths (e.g., 2-10 ms for TE values - does not have to be uniform over the whole range). Soft parameters may possibly be binned for a finite number of possible values to aggregate closely related scans and record them as one in the G3S. If exact values were taken, the index of table (A.3) 506 could explode because very similar scans would be recorded as new ones.

### Dimensionality reduction:

Once added to (A.3) 506, an MRI scan may be represented by a point in the high-dimensional G3S. As more scans are collected in the database, dimensionality reduction techniques can be applied (e.g., singular value decomposition (SVD), principal component analysis (PCA), or AI-based autoencoders) to reduce the dimensionality of the G3S, creating the G3S2, which can be transformed back to the G3S via the basis vectors provided by the dimensionality reduction technique. This leads not only to a compressed data load in the database (A.3) 506, but it may also offer new insights about MRI scan relationships based on actual clinical usage (see Fig. 3 for illustration). This may facilitate a data-based metric which relates different sequence types to each other and is filterable by body parts and even patient indications. This could be used to identify "blind spots" and might trigger more systematic developments of new sequences by studying voids in this abstract, lower-dimensional G3S2 and creating new sequences simply by placing new points into voids.

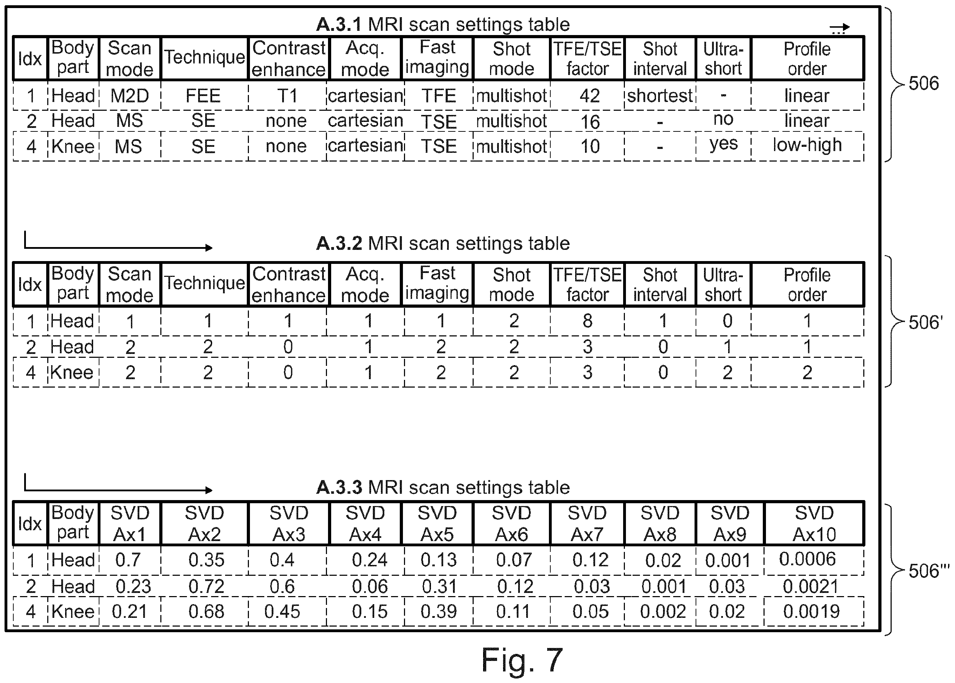

Fig. 7 shows several examples of the MRI scan setting table 506, 506', 506". At the top of the Figure a MRI scan setting table (A.3.1) 506 is shown that is identical with that illustrated in Fig. 6. In this scan table 506, there are a number of specifications, which are in text or in the form of a description. For example, there is an acquisition mode; this is a Cartesian for all three and also a shot mode as well as a mode for fast imaging. To enable more complicated mathematical methods, these various quantities may be encoded using numbers. The MRI scan setting table (A.3.2) 506' shows the same MRI scan setting table 506 after numerical encoding has been performed. The conversion of the scan settings into a purely numerical form may enable the use of dimensionality reduction and soft parameters may be binned into sensible ranges to coarse grain settings. This can be part of a hardware transformation function or HTF, which translates system-specific settings to the Generalized Settings Space (G3S), as illustrated in table 506'.

Once enough data has been collected, the features in the G3S table 506' can be recombined in a basis change. This new basis change may have vectors, for example SVD axes that are linear combinations of the original sequence features in the G3S and can be sorted, for example, according to decreasing degree of variance through all the different scans, for example, using principal component analysis. This may reveal new relations between different magnetic resonance imaging sequences and settings, possibly adding new sequences or aiding new sequence development. The MRI scan setting table (A.3.3) 506" illustrates the table 506' after basis change has been performed. Here, a singular value decomposition provides the SVD axes as a new basis. Starting from clear text for illustration (A.3.1) 506, each settings parameter is assigned a numeric, discrete value by the HTF to map a scan into the G3S representation (A.3.2) 506'. Dimensionality reduction can be used to leverage relationships and constraints between the settings and reduce the number of "independent" settings parameters, spanning the (ideally) lower-dimensional G3S2 (A.3.3) 506".

### B) Database usage: when a new patient needs an MRI exam.

i. A new patient prescribed with an MRI scan may possibly have a reason for this given by the prescribing physician. The reason(s) are extracted during or upon patient registration at the MRI scan site. Depending on the state of the graph database, this may be a medical code or several such (e.g., ICD-10), or word-based conditions, extracted from the prescribing doctor's report based on matches with the hash list maintained in (A,1). An NLP-AI can be used to support this extraction and add flexibility for wording and synonyms. The extracted symptoms are converted to a hash code combination through a lookup algorithm using table (A. 1) 504.
ii. The index of the graph database (A.2) 508 is searched for the attained hash code combination.
ii.a) If found:
   The list of references to MRI scans previously conducted for these symptoms (third column of table (A.2) 508 in Fig. 6) is used by the MRI protocol advisor tool to suggest possible MRI protocols for the new patient. Details on how this can be done are given in the next subsection (iii).
ii.b) If not found:
   Hash codes with highest similarity to the sought symptoms combination can be taken into account, e.g., by removing individual symptoms and searching for matches in the (A.2) 508 index (or searching for the symptoms individually). A list of references to MRI scans prescribed for similar symptoms or a subset thereof can be compiled this way and used to suggest scans for the new patient. In the worst case, the advisor tool cannot suggest any scans if no useful matches are found. Then, manual protocoling is necessary, as in current practice. The new protocol can be added to the database for the new symptoms combination as described in the previous section.
iii. The prior MRI sequence information extracted from the graph database in step (B.ii) serves as input for the MRI protocol advisor algorithm, possibly supported by AI. This is an interchangeable part of the invention, which is expected to become more sophisticated as the database and experience with it grow. The reference weights from table (A.2) 508 can aid in ranking the sequence suggestions. Also, some relevant patient information (like the presence of implants) and locally available hardware and methods constraints (like availability of contrast agent) could be included as input in order to exclude incompatible sequences). The scan advisor can be designed to mimic a prior scan card or, e.g., to span maximum distances in the G3S(2) with minimal scans to create a new protocol with high contrast diversity. Depending on the stage of the database, this may look as follows:
iii.a) Early stage (no dimensionality reduction yet in (A.3) 506 - just high-dimensional G3S):
   Each prior scan input consists of a coordinate in the G3S, with dimensions representing each scanner setting (translated to numeric values and binned where appropriate). For an on-site MRI, these coordinates can be translated to scanner-specific settings by the inverse HTF. This provides a list of MRI exams that could be conducted for this patient, with all sequence-related settings theoretically filled in (soft parameters roughly). These can be filtered according to local hardware and patient constraints, chosen by staff, and used as an exam card prior to be fine-tuned by staff, or algorithms in the future, to resolve conflicts and further adjust parameters to local conditions, patient particularities, and diagnostic objectives. AI models trained with the unbinned contents of (A.2) 508 could additionally fine-tune the rough settings for different indications (with continuous model training as new exams are added to the database). The final choice of which exam to choose from the available list may possibly be made by the staff at this point (possible remotely), but could later also be automated with usage frequency and diagnostic quality metrics as recorded in (A.2) 508 and (A.3) 506.
iii.b) Later stage (dimensionality reduction in place for (A.3) 506 - using lower-dimensional G3S2):
   In this case, each prior scan obtained from (B.ii) is a point coordinate in the lower-dimensional G3S2. Using the basis vectors of the dimensionality reduction method, the point coordinates in the G3S2 are transformed back to G3S and, using the inverse HTF, specialized for the specific scanner setup. It can then be used in analogy to (B.iii.a) to deliver a list of possible exams, initialized with prior settings that can be further fine-tuned. In the case that a prior scan could not be translated to the present system due to hardware or software incompatibility, the compressed G3S2 and its derived distance metric could be used to find "proximal" scan settings that may deliver similar contrasts or insights for the present patient (as an optionally enabled "discovery mode"). This possibility may possibly need further research but may also deliver new insights into how different contrasts are related for different tissues and pathologies, potentially fostering the development of new diagnostic gold standards or further optimization of sequences for specific purposes/questions.

The MRI protocol advisor 502 and access to the graph database 122 can be offered in a cloud-based software service. This allows for continuous updates and easy deployment of new features, usage monitoring, and continuous contributions to grow.

Beyond an MRI protocol advisor 502, the graph database 122 could enable many applications, some of which may currently be outside of our scope and imagination. The advantage of this approach of building up a database which holds connections between physiological symptoms, detailed MRI scan information, and possibly associated diagnoses and other patient information, is that it only represents a way of collecting and structuring the relevant information clearly for later use and analysis. The graph database can be used to extract multiple subgraphs, allowing for different filtering and separate focus on intra- and inter-graph connections, meaning connections within a specific subgraph and between the different subgraphs, respectively.

The way these connections are analyzed and used can be adapted as new technologies and algorithms are developed. Just some examples include:
- Studying individual subgraphs, which can be distilled from this database. For example, one can use the symptoms combination hash codes to extract how often individual symptoms generally occur together. A hyper-graph can easily be built from this, with nodes representing individual symptoms (node "size" indicating how often that symptom occurred in general) and hyper-edges between these nodes indicating simultaneous occurrence (edge weights indicating frequency).
- Index permutation: Parsing the database (A.2) 508 to create separate graph databases in analogy to (A.2) 508, but with a different searchable index (e.g., based on MRI scan combinations or diseases instead of symptoms). Such a reorganization would list different symptoms for each MRI scan combination or for each diagnosed disease (combination), offering new views on the data and new filtering options.
- Using graph-theoretic methods, like clustering and shortest paths analyses, to study relationships between different symptoms and MRI scans, within the subgraphs and between them.
- Continuous delivery: developing AI for the scan suggestion process which uses this database as a prior. Constant development during production may possibly be no problem with the described architecture.
- Post-market surveillance: acquire detailed information about scanner usage in different fields in a well-structured, easily searchable way. Automated methods and algorithms can be developed to extract information from the database which may aid development and refinement of sequences and protocols. This may possible enable cross-sequence protocol acceleration, e.g., by combining contrasts that are often used together to one optimized acquisition with joint reconstruction, information sharing and total protocol acceleration.
- Predictive maintenance: With the collected scanner usage information, sequences that would deliver often problematic image quality, e.g., due to bad signal-to-noise ratio or artefacts, could be recognized in an automated way. This could happen through the one-click feedback optionally given during image viewing by the radiology staff and letting a surveillance algorithm flag sequences in table (A.3) 506 with unacceptable complaint rating rates. Also, Al-supported algorithms could automatically rate images based on artefacts, noise, and/or other image quality metrics. The ratings of resulting images could be logged in the database (A.3) 506 and checked by surveillance algorithms.

A big advantage of this strategy of indexing of (A.3) 506 and referencing in (A.2) 508 is that it allows one to extract massive information about jointly used MRI sequences, filterable by symptoms and/or body parts. Such graph data is valuable for vendors and manufacturers and can guide the design of future, standardized scan cards for different purposes and body parts. One could answer questions like: which combination of neurological scans answers most (e.g., 90% of) clinic questions in neurology? Also, this makes the database very suitable to use for automated full examination suggestions, instead of just suggesting individual scans for different symptoms, as in previous proof-of-principles.

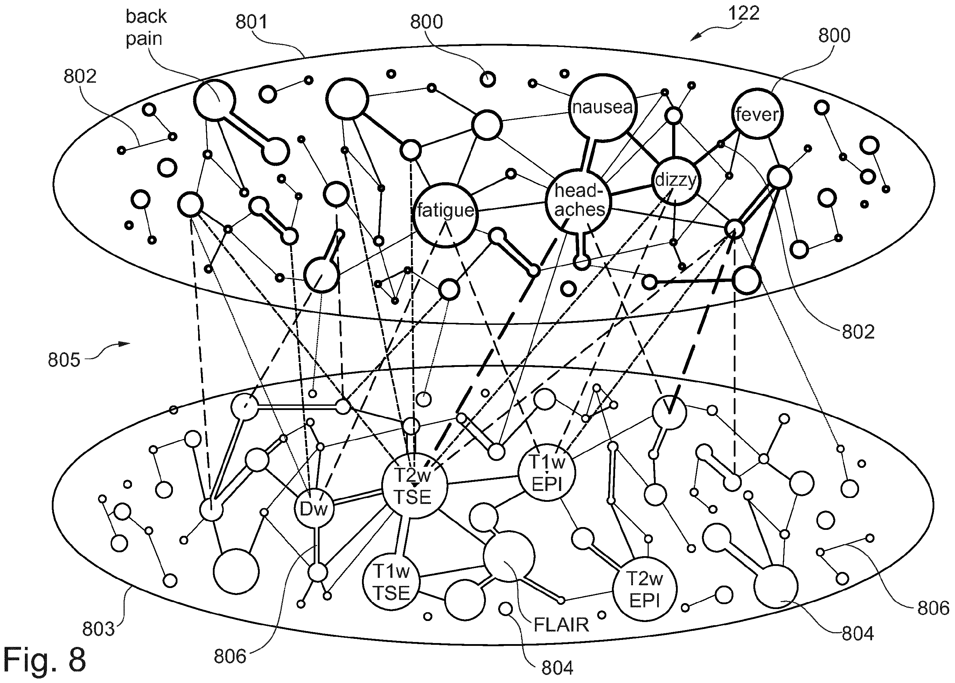

Fig. 8 illustrates the graph database 122 in graphical form. The subject hypergraph 801 is shown as comprising subject nodes 800 that are connected by subject hyperedges 802. The pulse sequence configuration hypergraph 803 is shown as comprising configuration nodes 804 that are connected by configuration hyperedges 806. The configuration nodes 804 represent individual pulse sequences and the configuration hyperedges 806 represent collections of pulse sequences or pulse sequence protocols. This may for example be a set of magnetic resonance imaging acquisitions that are performed together.

Fig. 8 contains a conceptual illustration of two subgraphs 801, 803 extracted from the envisioned database. At the top 801, the symptoms subgraph is sketched by representing nodes as circles (size corresponding to number of occurrences) and "intra-graph" edges as lines (thickness corresponding to edge weight, i.e., the number of mutual occurrences). More precisely, the intra-graph edges are hyperedges, generally connecting several nodes . In a second cloud below 803, the MRI sequence subgraph is illustrated. Here, individual nodes correspond to individual MRI scans as recorded in table (A.3) 506, but they may also be clustered sequence groups if some clustering was applied. The dotted lines between the subgraphs represent inter-graph edges 805 with different weights (thickness). The inter-graph connections can be extracted from the database to either connect individual nodes (scans from (A.3) 506 and symptoms from (A. 1) 504) or entire scan combinations with full symptoms combinations (essentially connections between hyper-edges from different subgraphs). Clustering algorithms can coarse-grain both subgraphs and the inter-graph connections, revealing different information depending on the clustering method.

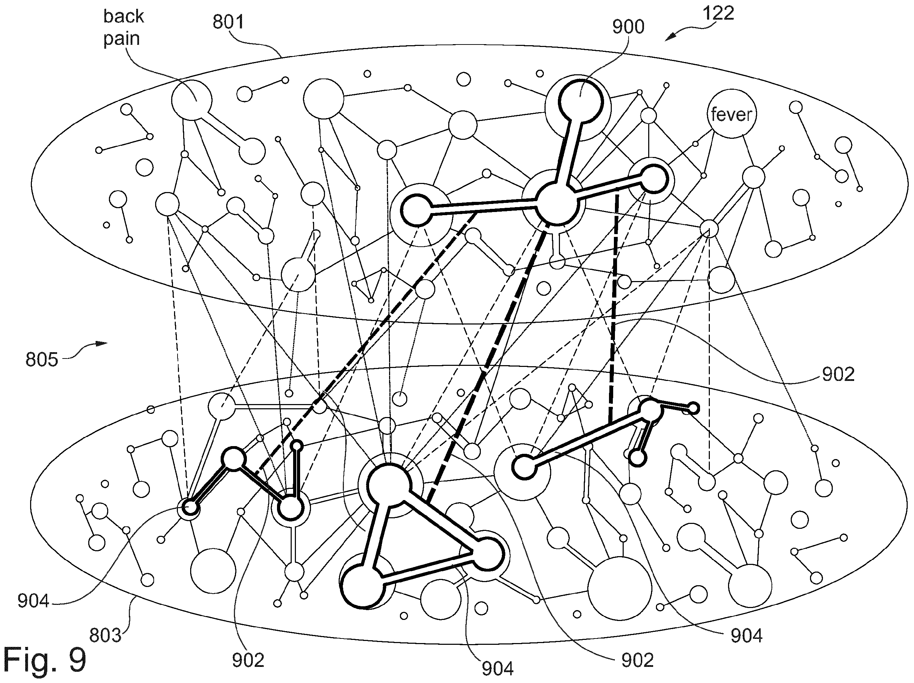

Fig. 9 shows an additional view of the graph data base 122. In this Fig., a search for the closest matching subject hyperedge 900 has been performed and is emphasized. The dark dashed lines 902 represent connections between the closest matching subject hyperedge 900 and configuration hyperedges with weighting factors. The thickness of the dashed lines represents a higher weighting factor. In the pulse sequence configuration hypergraph 803, the connections 902 go to several retuned configuration hyperedges 904.

Furthermore, dimensionality reduction of the G3S could offer new ways of systematic development of new sequences for specific symptoms/illnesses by studying neighborhoods and voids in the abstract sequence settings space G3S2. One could optimize coils for specific settings ranges as they are actually used in clinics.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

- 100: medical system
- 102: computer
- 104: magnetic resonance imaging system
- 106: magnetic resonance imaging system
- 108: magnetic resonance imaging system
- 110: computational system
- 112: user interface
- 112': user interface
- 114: network interface or hardware interface
- 114': network interface or hardware interface
- 116: memory
- 116': memory
- 120: machine executable instructions
- 122: graph database
- 124: subject data
- 126: magnetic resonance imaging pulse sequence configuration data
- 200: receive the subject data
- 202: receive the magnetic resonance imaging pulse sequence configuration data in response to inputting the subject data into the graph database
- 302: magnetic resonance imaging system
- 304: magnet
- 306: bore of magnet
- 308: imaging zone
- 309: field of view
- 310: magnetic field gradient coils
- 312: magnetic field gradient coil power supply
- 314: radio frequency coil
- 316: transceiver
- 318: subject
- 320: subject support
- 330: survey scan pulse sequence commands
- 332: survey scan k-space data
- 334: survey scan data
- 336: local subject data
- 338: image classification machine learning module
- 340: image classification
- 342: one or more pulse sequences
- 344: k-space data
- 346: one or more magnetic resonance images
- 400: receive magnetic resonance imaging survey scan data descriptive of the subject;
- 402: derive at least a portion of the subject data from the survey scan data.
- 404: receive a selection of a magnetic resonance imaging system
- 406: translate the received magnetic resonance imaging pulse sequence configuration data to one or more pulse sequences configured to control the magnetic resonance imaging system to acquire k-space data of the subject
- 408: acquire the k-space data of the subject by controlling the one or more magnetic resonance imaging system with the one or more pulse sequences
- 410: reconstruct one or more magnetic resonance images from the k-space data
- 500: medical system
- 502: magnetic resonance imaging protocol adviser
- 504: subject hypergraph
- 506: pulse sequence configuration hypergraph
- 508: main graph data frame
- 510: decision module
- 512: approval feedback mechanism
- 514: retry subset of symptoms
- 516: manual protocoling
- 518: add new pulse sequences protocols to database
- 800: subject nodes
- 801: subject data hypergraph
- 802: subject hyperedges
- 803: pulse sequence configuration hypergraph
- 804: configuration nodes
- 805: connections between subject hyperedges and configuration hyperedges
- 806: configuration hyperedges
- 900: closest matching subject hyperedge
- 902: connections between the closest matching subject hyperedge and configuration hyperedges with weighting factors
- 904: returned configuration hyperedges

## Claims

1. A medical system (100, 300, 500) comprising:
- a memory (116) storing machine executable instructions (120) and a graph database (122), wherein the graph database is configured to output magnetic resonance imaging pulse sequence configuration data (126) in response to receiving subject data; and
- a computational system (110), wherein execution of the machine executable instructions causes the computational system to:
- receive (200) the subject data; and
- receive (202) the magnetic resonance imaging pulse sequence configuration data in response to inputting the subject data into the graph database.

2. The medical system of claim 1, wherein the graph data base comprises a subject data hypergraph (801) and a pulse sequence configuration hypergraph (803),
wherein the subject data hypergraph comprises subject nodes (800) encoding the subject data (504), wherein the subject data hypergraph further comprises subject hyperedges (802) connecting the subject nodes and encoding joint occurrences of the subject data,
wherein the pulse sequence configuration hypergraph comprises configuration nodes (804) encoding individual configuration data for an individual magnetic resonance imaging pulse sequence (506), wherein the pulse sequence configuration hypergraph further comprises configuration hyperedges (806) which connect the configuration nodes to encode pulse sequence combinations forming the magnetic resonance imaging pulse sequence configuration data, and
wherein the database further comprises a main graph data frame (508) listing each subject hyperedge and connections (805) to configuration hyperedges.

3. The medical system of claim 2, where the connections to the configuration hyperedges and the configuration hypergraph nodes comprise weighting factors, and wherein the graph database is configured to select the magnetic resonance imaging pulse sequence configuration data by using the subject data and weighting data comprising:
- identifying a closest matching subject hyperedge (900) in the subject data hypergraph using the subject data;
- returning configuration hyperedges (904) and weighting data (902) associated with the closest matching subject hyperedge in the main graph data frame; and
- selecting the magnetic resonance imaging pulse sequence configuration data from the returned configuration hyperedges at least partially using the weighting data.

4. The medical system of claim 3, each subject data hyperedge being identified by a unique hash number, wherein execution of the machine executable instructions further comprises:
- converting the subject data into a subject hash number; and
- searching for the closest matching subject hyperedge in the main graph data frame using the subject hash number.

5. The medical system of any one of claims 2 through 4, wherein the encoding of the magnetic resonance imaging pulse sequence configuration data for an individual pulse sequence is numerical.

6. The medical system of claim 5, wherein the graph database is configured to add new configuration nodes to the configuration hypergraph by binning pulse sequence parameters.

7. The medical system of claim 5 or 6, wherein the method further comprises:
- calculating a distance within the magnetic resonance imaging pulse sequence configuration data for the configuration nodes connected by the configuration hyperedges associated with the closest subject hyperedge in the main graph data frame; and
- selecting the magnetic resonance imaging pulse sequence configuration data from the returned configuration hyperedges by using a combination of the weighting data and the calculated distance with the magnetic resonance imaging pulse sequence configuration data.

8. The medical system of claim 7, wherein the configuration nodes encode pulse sequence settings in a reduced dimensionality state, wherein the memory further comprises a mapping between the reduced dimensionality state and magnetic resonance imaging system pulse sequence configuration parameters, wherein providing the magnetic resonance imaging pulse sequence configuration data comprises converting between the reduced dimensionality state and the magnetic resonance imaging system pulse sequence configuration parameters.

9. The medical system of any one of the preceding claims, wherein execution of the machine executable instructions further causes the computational system to:
- receive (400) magnetic resonance imaging survey scan data (334) descriptive of the subject;
- derive (402) at least a portion of the subject data from the survey scan data.

10. The medical system of claim 9, wherein deriving the at least a portion of the subject data from the survey scan data comprises:
- determining an image segmentation of the survey scan data using an automatic segmentation module; and
- calculating the at least a portion of the subject data using the image segmentation.

11. The medical system of claim 10, wherein the memory further comprises an image classification machine learning module, wherein deriving the at least a portion of the subject data comprises:
- receiving an image classification in response to inputting the survey image data into the image classification machine learning module; and
- providing the image classification as the at least a portion of the subject data.

12. The medical system of any one of the preceding claims, wherein execution of the machine executable instructions further causes the computational system to:
- receive (404) a selection of a magnetic resonance imaging system;
- translate (406) the received magnetic resonance imaging pulse sequence configuration data to one or more pulse sequences configured to control the magnetic resonance imaging system to acquire k-space data of the subject.

13. The medical system of claim 12, wherein the medical system further comprises one or more magnetic resonance imaging systems (104, 106, 108), wherein execution of the machine executable instructions further causes the computational system to acquire (408) the k-space data of the subject by controlling the one or more magnetic resonance imaging system with the one or more pulse sequences.

14. A method of operating a medical system (100, 300, 500), wherein the medical system comprises a computational system (110), wherein the method comprises:
- receiving (200) the subject data; and
- receiving (202) magnetic resonance imaging pulse sequence configuration data (126) in response to inputting the subject data into a graph database (122), wherein the graph database is configured to output magnetic resonance imaging pulse sequence configuration data in response to receiving the subject data.

15. A computer program comprising machine executable instructions (120) for execution by a computational system (110), wherein execution of the machine executable instructions causes the computational system to:
- receive (200) the subject data; and
- receive (202) the magnetic resonance imaging pulse sequence configuration data in response to inputting the subject data into a graph database (122), wherein the graph database is configured to output magnetic resonance imaging pulse sequence configuration data in response to receiving the subject data.
